# EUROPÄISCHE PATENTANMELDUNG

(11) **EP 1 344 521 A1**
(43) Veröffentlichungstag der Anmeldung: **17.09.2003**
(21) Anmeldenummer: 03002948.2
(22) Anmeldetag: 11.02.2003
(51) Int. Cl.: A61K 9/19

(54) **Verfahren zur Rekonstitution von lyophilisierten Proteinen**

(30) Priorität: 13.03.2002 DE 10211227
(71) Anmelder: Aventis Behring GmbH, 35002 Marburg (DE)
(72) Erfinder: Rapp, Mirna, 35037 Marburg (DE); Grandgeorge, Michel, 69670 Vaugneray (FR)

(57) **Zusammenfassung**

Es wird ein Verfahren zur Rekonstitution von lyophilisierten Proteinen beschrieben, bei dem die Rekonstitution in einem Behälter erfolgt, in dem ein Gasdruck zwischen 1mbar und Atmosphärendruck herrscht. In dem das lyophilisierte Protein enthaltenden Behälter wird der erfindungsgemäß erforderliche Gasdruck zwischen 1 mbar und Atmosphärendruck durch Einleiten von Luft oder eines Inertgases eingestellt und die zur Auflösung des Proteins erforderliche Menge Wasser für Injektionszwecke hinzugegeben. Das Verfahren ist insbesondere für Blutgerinnungsfaktoren, den von Willebrand-Faktor und Albumin geeignet.

## Beschreibung

Gegenstand der Erfindung ist ein Verfahren zur Rekonstitution von lyophilisierten Proteinen, bei dem ein optimales Auflöseverhalten des Proteins durch Einstellung bestimmter Druckverhältnisse bei der Rekonstitution gewährleistet wird.

Es ist üblich, als Arzneimittel eingesetzte Proteine durch eine als Lyophilisation bezeichnete Gefriertrocknung in eine über längere Zeit lagerfähige Form zu überführen und dabei ihre biologische Wirksamkeit zu erhalten. Bei der Gefriertrocknung werden die labilen Proteine unter sterilen Bedingungen schnell und schonend eingefroren und anschließend das in Eis umgewandelte Wasser durch Sublimation unter Hochvakuumbedingungen schnell entfernt. Durch die Verdunstungskälte bleibt dabei die zu trocknende Substanz im gefrorenen Zustand.

Bei der praktischen Herstellung von Lyophilisaten werden üblicherweise die das Protein enthaltenden Lösungen in kleine, zur Abgabe vorgesehene Behälter eingefüllt, sogenannte Lyophilisationsstopfen aufgesetzt, und in eine Gefriertrocknungskammer eingeführt. Noch auf den Stellflächen werden die Gefäße dann durch ein hydraulisches oder manuelles Zusammendrücken der Stellflächen in der Gefriertrocknungskammer verschlossen, wobei die Stopfen in die Gefäße gedrückt werden. Dadurch ist gewährleistet, dass sich in den Behältern ein vergleichbares Vakuum wie in der Gefriertrocknungskammer befindet. Anschließend wird die Kammer belüftet, die Behälter mit den fertigen Lyophilisaten entnommen, die anschließend mit Bördel-Kappen verschlossen und gebrauchsfertig verpackt werden.

Dieses Verfahren wird im großen Umfang z. B. für die Blutgerinnungsfaktoren, darunter die Faktoren VIII, von Willebrand-Faktor (vWF) sowie Faktor IX (F IX) eingesetzt. Die Lyophilisate können zur Stabilisierung Salze, Mono- und Polysaccharide, organische Polymere und andere Proteine wie Albumin enthalten. Die lyophilisierten Proteine können einzeln oder kombiniert in Fertigprodukten zum Beispiel zur Behandlung der Hämophilie A oder der von Willebrand-Erkrankung verabreicht werden. Kombinationen des Blutgerinnungsfaktors VIII und des von Willebrand-Faktors können zur Behandlung der von Willebrand-Erkrankung ohne eine zusätzliche Verabreichung von Faktor VIII eingesetzt werden. Im Falle der Behandlung der Hämophilie A erwies sich ein höherer Anteil an von Willebrand-Faktor als vorteilhaft zum Schutz vor der möglichen Bildung von Inhibitoren gegen den Blutgerinnungsfaktor VIII. Ein derartiges Kombinationspräparat wird nach der Herstellung einer Mischung beider Komponenten in Glasfläschchen abgefüllt und nach einem bestimmten Programm lyophilisiert. Nach erfolgter Gefriertrocknung wird das Lyophilisat noch unter Vakuum verschlossen. Das Vorhandensein eines Unterdrucks im Behälter ist eine Voraussetzung für den Auflöseprozess unmittelbar vor dem Aufziehen der Wirkstofflösung in die Spritze. Das Lyophilisat wird dabei in einen physiologisch verträglichen wässrigen Lösungsmittel, z. B. PBS (phosphate buffered saline) oder Wasser für Injektionszwecke (WFI) aufgelöst, welches sich in einem separaten Glasgefäß befindet. Mit Hilfe eines Überleitungssystems wird das Lösungsmittel in das Gefäß mit dem Lyophilisat überführt. Dadurch erfolgt ein rasches Einfließen des Lösungsmittels unter möglichst keimfreien Bedingungen.

Es wurde nun die Beobachtung gemacht, dass ein sehr niedriger Vakuumwert zwar ein rasches Einfließen des WFI in den Behälter bewirkt, jedoch einen nachteiligen Einfluss auf die Löslichkeit des Lyophilisats haben kann. Wenn in dem Arzneimittelfläschchen ein zu starker Unterdruck herrscht, kann das zur Folge haben, dass die Auflösung von kleinen Anteilen des Lyophilisats so weit verzögert wird, dass die vorgesehene Dauer für eine vollständige Auflösung des Proteins überschritten wird. Obwohl sich fast der gesamte Anteil des Lyophilisats rasch auflöst, bleiben in manchen Fällen doch noch Lyophilisatreste übrig, die an der Oberfläche der wässrigen Lösung schwimmen. Sie sind sehr leicht und lassen sich auch durch eine Zentrifugation bis zu 14.000 rpm nicht sedimentieren. Mikroskopisch gesehen enthalten sie viele eingeschlossene winzige Luftbläschen. Diese Bläschen sind wahrscheinlich eine Folge von Luft- oder Wasserdampf-Einschlüssen während der Rekonstitution bei sehr hohem Vakuum. Diese schwer löslichen Lyophilisatreste zeigen eine so verzögerte Auflösung, dass in manchen Fällen bis zu 4 Stunden bis zur vollständigen Auflösung vergehen. Als vollständige Auflösung wird die Abwesenheit optisch erkennbarer nicht sedimentierbarer Partikel angesehen. Die biochemische Analyse ergibt, dass diese schwer löslichen Partikel eine identische chemische Zusammensetzung wie das gelöste Material haben und auch keine weiteren Fremdpartikel oder Abbauprodukte enthalten. Die schwer löslichen Lyophilisatreste sind im Durchschnitt 2 mm groß, stellen aber keine Gefahr für den Patienten dar, da die in den Behälter hergestellte Lösung stets über ein in der Packung befindliches Filtersystem geleitet wird und dadurch ungelöste Anteile entfernt werden. Das trifft auch für andere ungelöste Anteile, sog. fuselförmige Partikel zu, bei denen es sich um mikroskopisch kompakte und daher sedimentierbare Teilchen handelt, die nach dem Auflösen von von Willebrand-Faktor-haltigen Präparaten gelegentlich beobachtet werden.

Es stellte sich deshalb die Aufgabe, das Rekonstitutionsverfahren von lyophilisierten Proteinen zu verbessern und ein schnelles und vollständiges Auflösen zu erreichen. Ansatzpunkt für die schließlich gefundene Lösung war dabei die Beobachtung, dass die Zugabe von Wasser für Injektionszwecke in einem Vakuum-freien Behälter zu einer vollständigen Auflösung des gesamten Lyophilisatkuchens innerhalb von 1 bis 2 Minuten führt. Hieraus wurde ein Zusammenhang zwischen dem Vakuum in dem das Lyophilisat enthaltenden Behälter und dem Auftreten von Partikeln mit verzögerten Auflöseverhalten erkannt. Weitere Untersuchungen haben dann gezeigt, dass der in dem Lyophilisatbehälter zu Beginn der Rekonsitution herrschende Gasdruck entscheidenden Einfluss auf die Auflösung des lyophilisierten Proteins hat.

Bei diesen Untersuchungen wurde ein Verfahren zur Rekonstitution von lyophilisierten Proteinen gefunden, bei dem die Rekonstitution in einem Behälter erfolgt, in dem ein Gasdruck zwischen 1 mbar und Atmosphärendruck herrscht. Günstig ist ein Verfahren, bei dem die Rekonstitution in einem Behälter erfolgt, in dem ein Gasdruck zwischen 100 mbar und Atmosphärendruck herrscht. Besonders günstig ist ein Verfahren bei dem die Gasdrucke zwischen 100 und 300 mbar herrscht. Unter diesen Bedingungen ist das Auftreten von schwer löslichen Partikeln (Lyophilisatresten) mit großer Sicherheit vermeidbar. Bei den genannten Druckbedingungen ist eine rasche Überführung des Lösungsmittels, also z. B. des Wassers für Injektionszwecke, in den Lyophilisatbehälter ebenso gut möglich wie bei Vakuumwerten unterhalb von 1 mbar.

Das erfindungsgemäße Rekonstitutionsverfahren für Proteine erfordert, dass in dem das Lyophilisat enthaltenden Behälter kein Hochvakuum mehr herrscht. Im allgemeinen wird eine Gefriertrocknung so durchgeführt, dass während des gesamten Verlaufs bei konstanten Druck- und Temperaturbedingungen gearbeitet wird, sobald der Einfriervorgang abgeschlossen ist. Meist läuft aber eine Gefriertrocknung von empfindlichen Materialien wie den Plasmaproteinen nach einem genau definierten Programm, bei dem die Temperatur der Stellflächen in der Gefriertrocknungskammer nach bestimmten Zeitinterwallen schrittweise angehoben wird. Ebenso können die Vakuumbedingungen variiert werden, um einen gewünschten Restfeuchtegehalt im Lyophilisat nach erfolgter Gefriertrocknung aufrecht zu erhalten.

Erfindungsgemäß wird deshalb nach der im Hochvakuum erfolgten Lyophilisation in dem das Protein enthaltenden Behälter ein Gasdruck zwischen 1 mbar und Atmosphärendruck, vorzugsweise zwischen 100 mbar und Atmosphärendruck, durch Einleiten von Luft oder einem Inertgas eingestellt und zur Auflösung des Proteins in den Behälter die erforderliche Menge Wasser für Injektionszwecke gegeben.

Die Einstellung auf einen bestimmten Vakuumwert kann sowohl durch eine entsprechende Änderung der Programmbedingungen als auch durch eine zeitweise Belüftung der Trocknungskammer erfolgen. Eine Belüftung kann mittels zugeführter Luft oder mittels Inertgasen wie Stickstoff erfolgen. Da lyophilisierte Produkte üblicherweise unter sterilen Bedingungen verschlossen werden müssen, wird das zugeführte Gas über ein Sterilfilter geleitet.

Weiterhin ist ein Gegenstand der Erfindung ein Kit zur Bereitstellung einer injezierbaren Lösung mindestens eines therapeutischen Proteins, wobei der Kit einen Behälter enthält, der das oder die therapeutischen Protein(e) umfasst, in dem ein höchstens bis auf 1mbar verminderter Luftdruck herrscht, wobei der Behälter ganz oder teilweise auch mit einem Schutzgas, wie z.B. Stickstoff, befüllt sein kann. Günstig ist es, wenn der Luftdruck in dem Behälter zwischen 100 und 300 mbar liegt. Der erfindungsgemäße Kit kann auch zusätzlich einen Behälter mit einem physiologischen Lösungsmittel enthalten. Das verwendete therapeutische Protein, das z.B. ein Blutgerinnungsfaktor, insbesondere Faktor VIII, sein kann, kann natürlichen Ursprungs sein oder rekombinant gewonnen worden sein. Ebenfalls können Mutanten oder Teilsequenzen von Blutgerinnungsfaktoren als therapeutische Proteine im Sinne der Erfindung eingesetzt werden.

Die Erfindung wird in den nachfolgenden Beispielen näher erläutert.

### Beispiel 1

Die Rekonstitution von Haemate HS / Humate P (Aventis Behring, Marburg, Deutschland) mit Abfüllungen, die im Hochvakuum verschlossen werden, wurde mit Abfüllungen ohne Vakuum (Atmosphärendruck) verglichen. Das Lösungsmittel (WFI) wurde mit dem Überleitsystem bzw. mit der Einmalspritze in das Gefäß mit dem Lyophilisat überführt. Das Ergebnis zeigte in allen Fällen eine vollständige Auflösung des Lyophilisates unter Atmosphärendruck. Abfüllungen mit Hochvakuum zeigten in einigen Fällen eine verzögerte Auflösung von kleinen Lyophilisatresten über 10 Minuten hinaus. Die Geschwindigkeit der Applikation von WFI war nicht maßgebend für das Auftreten der schwer löslichen Lyophilisatreste.

### Beispiel 2

Lyophilisierte Abfüllungen von Haemate wurden bei verschiedenen Vakuum-Werten in der Gefriertrocknungsanlage verschlossen. Die Rekonstitution mit WFI erfolgte mittels dem für Haemate HS / Humate P zugelassenen Überleitsystems. Dokumentiert wurde die Zeit des Einfließens von WFI sowie das Auftreten der schwer löslichen Lyophilisatreste. Wie in Tabelle 1 dargestellt, ist ein Vakuumwert von größer als 100 mbar für die vollständige Rekonstitution des Lyophilisates vorteilhaft. Bei diesen Druckbedingungen ist noch eine rasche Überführung des Lösungsmittels möglich.

### Tabelle I:

Rekonstitutionsverhalten von Haemate HS / Humate P in den Abfüllungen mit unterschiedlichen Vakuumwerten

| **Vakuum im Lyophilisatgefäß** | **Einfließzeit des Lösungsmittels** | **Beobachtung von schwer löslichen Lyophilisatresten** |
|---|---|---|
| 0.025 mbar | 11 sec. | Ja |
| 0.25 mbar | 13 sec. | Ja |
| 1 mbar | 13 sec. | Ja |
| 100 mbar | 13 sec. | ja/nein |
| 175 mbar | 14 sec. | nein |
| 250 mbar | 14 sec. | nein |
| | | |

Aus der Tabelle geht hervor, dass ein Vakuumwert über 100 mbar in den lyophilisierten Abfüllungen einen deutlichen Vorteil für die Rekonstitution mit dem Überleitsystem darstellt.

## Patentansprüche

1. Verfahren zur Rekonstitution von lyophilisierten Proteinen, **dadurch gekennzeichnet, dass** die Rekonstitution in einem Behälter erfolgt, in dem ein Gasdruck zwischen 1mbar und Atmosphärendruck herrscht.

2. Verfahren nach Anspruch 1, **dadurch gekennzeichnet, dass** die Rekonstitution in einem Behälter erfolgt, in dem ein Gasdruck zwischen 100mbar und Atmosphärendruck herrscht.

3. Verfahren nach Ansprüchen 1 und 2, **dadurch gekennzeichnet, dass** nach der im Hochvakuum erfolgten Lyophilisation in dem das Protein enthaltenden Behälter ein Gasdruck zwischen 1mbar und Atmosphärendruck durch Einleiten von Luft oder einem Inertgas eingestellt und zur Auflösung des Proteins in den Behälter die erforderliche Menge Lösungsmittel gegeben wird.

4. Verfahren nach den Anspruch 3, **dadurch gekennzeichnet, dass** die zur Einstellung des Gasdruckes in dem Behälter verwendete Luft oder das Inertgas über ein Sterilfilter geleitet wird.

5. Verfahren nach den Ansprüchen 1 bis 4, **dadurch gekennzeichnet, dass** als Protein ein Plasmaprotein eingesetzt wird.

6. Verfahren nach den Ansprüchen 1 bis 5, **dadurch gekennzeichnet, dass** als Plasmaprotein ein Blutgerinnungsfaktor, der von Willebrand-Faktor oder Albumin eingesetzt wird.

7. Verfahren nach Ansprüchen 1 und 2, **dadurch gekennzeichnet, dass** nach der im Hochvakuum erfolgten Lyophilisation in dem das Protein enthaltenden Behälter ein Gasdruck zwischen 1mbar und Atmosphärendruck durch entsprechende Wahl der Programmbedingungen in der Gefriertrocknungsanlage eingestellt und zur Auflösung des Proteins in den Behälter die erforderliche Menge Wasser für Injektionszwecke gegeben wird.

8. Kit zur Bereitstellung einer injezierbaren Lösung mindestens eines therapeutischen Proteins, **dadurch gekennzeichnet, dass** es einen Behälter enthaltend das oder die therapeutischen Protein(e) umfasst, in dem ein höchstens bis auf 1mbar verminderter Luftdruck herrscht.

9. Kit gemäß Anspruch 8 wobei der Behälter ein Schutzgas enthält.

10. Kit gemäß einem der Ansprüche 8 bis 9, wobei der Luftdruck in dem Behälter zwischen 100 und 300 mbar liegt.

11. Kit gemäß einem der Ansprüche 8 bis 10, wobei der Kit zusätzlich einen Behälter mit einem physiologischen Lösungsmittel enthält.

12. Kit gemäß einem der Ansprüche 8 bis 11, wobei das therapeutische Protein natürlicher oder rekombinant gewonnener Faktor VIII, oder eine Mutante von Faktor VIII ist.
